# EUROPÄISCHE PATENTANMELDUNG

(11) **EP 0 635 717 A2**
(43) Veröffentlichungstag der Anmeldung: **25.01.1995**
(21) Anmeldenummer: 94110903.5
(22) Anmeldetag: 13.07.1994
(51) Int. Cl.: G01N 27/12

(54) **Sensor mit einem in einem Gehaeuse angeordneten Sensorelement**

(30) Priorität: 22.07.1993 DE 4324659
(71) Anmelder: SIEMENS AKTIENGESELLSCHAFT, D-80333 München (DE)
(72) Erfinder: Gerblinger, Josef, Dr., D-86152 Augsburg (DE); Lampe, Uwe, Dr., D-21614 Buxtehude (DE); Meixner, Hans, Prof.-Dr., D-85540 Haar (DE)

(57) **Zusammenfassung**

2.1. Für die zylinderselektive λ-Regelung eignen sich insbesondere schnelle Gassensoren auf der Basis halbleitender Metalloxide. Diese Sensoren werden üblicherweise in sogenannten Visiergehäusen eingebaut, um die nur wenige µm dicken Metalloxidschichten vor den in Abgas mitgeführten Teilchen zu schützen.

2.2. Ziel der Erfindung ist die Schaffung eines Sensors, insbesondere eines Gassensors, dessen Gehäuse eine wirbelfreie Umströmung des Sensorelements gewährleistet und Ablagerungen auf den sensitiven Bereichen verhindert.

2.3. Das Sensorgehäuse (1) ähnelt im Aufbau einer Pfeife. Es besitzt einen s-förmig gekrümmten Strömungskanal, der eine Eintrittsöffnung (2) mit einer Austrittsöffnung (3) verbindet. Das planare Sensorelement (4) ist in einem weitgehend laminar durchströmten Abschnitt des Kanals hinter einer in die Eintrittsöffnung (2) mündenden Krümmung angeordnet. Da die in den gasförmigen oder flüssigen Medium mitgeführten schwereren Teilchen der Kanalkrümmung nicht folgen können, prallen sie auf die Kanalwand und bleiben dort haften. Die in der umgelenkten Strömung eventuell noch mitgeführten leichteren Teilchen werden von einem dem Sensorelement (4) vorgelagerten Metallsteg (5) abgefangen.

3. λ-Sonde

## Beschreibung

Das Betriebsverhalten eines Verbrennungsmotors hängt in entscheidender Weise von der Qualität der Gemischaufbereitung ab. So lassen sich die Schadstoffemission und der Kraftstoffverbrauch eines Motors durch eine dem jeweiligen Betriebszustand angepaßte Zumessung des Kraftstoffs zur Ansaugluft erheblich verringern. Dies gilt in besonderem Maße für einen mit einem geregelten Dreiwegekatalysator ausgestatteten Kfz-Verbrennungsmotor. Der der Reduzierung der Schadstoffemission dienende Katalysator arbeitet allerdings nur in einem sehr kleinen Luftzahlbereich mit einem hohen Wirkungsgrad. Um einen maximalen Konversionsgrad zu gewährleisten, darf das Luft/Kraftstoff-Verhältnis in jedem Betriebszustand des Motors daher nur um wenige Prozent von einem das jeweilige Optimum repräsentierenden Sollwert abweichen.

Die der Bestimmung der Luftzahl λ dienenden Sonden auf der Basis des bei höheren Temperaturen ionenleitenden Zirkonoxids sprechen vergleichsweise langsam auf Änderungen des Sauerstoffpartialdrucks im Abgas an. Sie eignen sich daher nicht zur zylinderselektiven λ-Regelung und werden in Zukunft vorteilhafterweise durch Metalloxid-Sensoren ersetzt, deren Ansprechzeit nur wenige Millisekunden beträgt. Diese in Planartechnik hergestellten Gassensoren besitzen üblicherweise einen aus einem keramischen Material bestehenden Grundkörper, auf dessen Oberfläche zwei Interdigitalelektroden und eine die Elektroden leitend verbindende Metalloxidschicht (z. B. SrTiO₃, CeO₂ oder Ga₂O₃) aufgebracht sind. Ein auf der Rückseite des Grundkörpers vorhandenes Widerstandselement erlaubt die aktive Beheizung des Gassensors. Strömt Sauerstoff über das thermisch aktivierte Metalloxid, so ändert sich dessen Widerstand bzw. Leitwert reversibel aufgrund komplizierter Adsorptionsprozesse an der Oxidoberfläche. Die Sauerstoffkon zentration im Abgas läßt sich daher in einfacher Weise durch eine Widerstands- bzw. Leitwertmessung bestimmen.

Ablagerungen auf der Oberfläche der nur wenige µm dicken Metalloxidschicht können deren gassensitive und elektrischen Eigenschaften erheblich beeinflussen. Sensoren auf der Basis halbleitender Metalloxide werden deshalb in einem Gehäuse angeordnet, um die im Abgas eines Verbrennungsmotors vorhandenen Partikel von den O₂-sensitiven Bereichen fernzuhalten. Die die Sensorfunktion beeinträchtigenden Partikel stammen aus den Additiven und den Verunreinigungen der Betriebsstoffe (Schmieröl, Benzin usw.) bzw. entstehen während des Motorbetriebs durch Abrieb. Sie sind typischerweise etwa 1 bis 2 µm groß und bestehen unter anderem aus Eisenoxiden.

Die US-A-4,916,934 beschreibt einen aus einem planaren Sensorelement und einem zylinderförmigen Gehäuse bestehenden Sauerstoffdetektor. Um ein direktes Anströmen der sauerstoffempfindlichen Schicht zu verhindern, sind das Sensorelement und die in der Mantelfläche des Gehäuses vorhandenen Gaseintrittsöffnungen in verschiedenen Ebenen angeordnet. Die den Eintrittsöffnungen jeweils zugeordneten Leitbleche sollen die im Abgas vorhandenen Partikel abfangen und eine zirkulare Gasströmung im Gehäuseinnern hervorrufen.

Das aus der EP-A-0 503 295 bekannte Zylindergehäuse für einen schnellen Abgassensor besitzt ebenfalls mehrere schlitzförmige Gaseintrittsöffnungen, deren Ränder sich nach Art einer Jalusie überlappen. Da die Schlitze als Partikelfallen wirken, werden Ablagerungen auf der empfindlichen Sensorschicht wirkungsvoll unterdrückt.

Um Ablagerungen in der aus DE 35 00 088 A1 bekannten Vorrichtung zum Nachweis von Chlor und chlorhaltigen Verbindungen zu verhindern, werden dem als Sensorelement dienenden Festelek trolyten nur Testgase zugeführt, die keine Partikel und Flüssigkeitstropfen enthalten. Der weitgehend unporöse und gedichtet in ein Gehäuse eingebaute Festelektrolyt trennt zwei jeweils zylindrische Kammern, wobei die eine Kammer mit dem Meßgas, die andere Kammer mit einem Referenzgas gefüllt ist. In die Kammern münden jeweils zwei Gehäusebohrungen, die den Gasaustausch ermöglichen.

Ziel der Erfindung ist die Schaffung eines Sensors, der einem partikelbelasteten Gas- oder Flüssigkeitsstrom längere Zeit ohne Schaden zu nehmen ausgesetzt werden kann. Das Sensorgehäuse soll den Gas- bzw. Flüssigkeitsaustausch mit dem Senscrelement nicht behindern und die
Lebensdauer der sensitiven Bereiche erhöhen. Diese Aufgaben werden erfindungsgemäß durch einen Sensor nach Patentanspruch 1 gelöst.

Der mit der Erfindung erzielbare Vorteil besteht insbesondere darin, daß das nahezu partikelfreie Gas weitgehend laminar und parallel zur Oberfläche des planaren Sensorelements strömt. Da im Kanal des Gehäuses keine Verwirbelung der von einem Verbrennungsmotor erzeugten Abgaspakete stattfindet, kann der mit einem schnellen sauerstoffempfindlichen Element ausgestattete Sensor als λ-Sonde zur zylinderselektiven Regelung der Luftzahl eingesetzt werden.

Die abhängigen Ansprüche betreffen vorteilhafte Weiterbildungen und Ausgestaltungen der im folgenden anhand der Zeichnung erläuterten Erfindung. Hierbei zeigt:
- Fig. 1: den schematischen Aufbau eines erfindungsgemäßen Gassensors,
- Fig. 2 und 3: Ausführungsbeispiele planarer Sensorelemente,
- Fig. 4 und 5: Heizelemente des Gassensors nach Fig. 3,
- Fig. 6 und 7: die Elektroden zur Kontaktierung der gasempfindlichen Metalloxidschicht des Sensorelements nach Fig. 3,
- Fig. 8 und 9: ein Ausführungsbeispiel eines Temperatursensors,
- Fig. 10: ein Ausführungsbeispiel des Sensorgehäuses im Schnitt;
- Fig. 11 und 12: den Kopf und den Grundkörper des Sensorgehäuses nach Fig. 10,
- Fig. 13: Schnitte durch das mit dem Sensorelement ausgestattete Gehäuse,
- Fig. 14: die den unteren Teil des Sensorgehäuses abschließende Keramikplatte,
- Fig. 15: die Verschaltung und die Anschlüsse der Sensorkomponenten,
- Fig. 16: ein mehrere Gaseintritts- und Gasaustrittsöffnungen aufweisendes Sensorgehäuse.

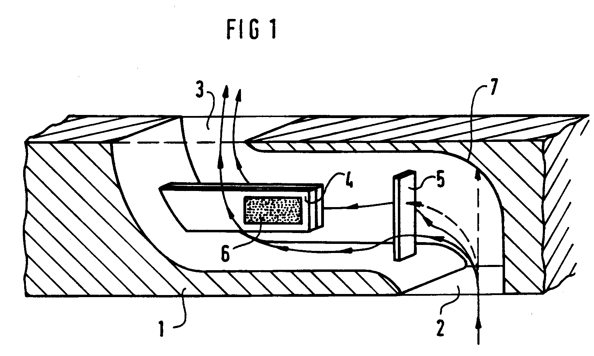

Das Stahlgehäuse 1 des in Fig. 1 schematisch dargestellten Gassensors ähnelt im Aufbau einer Pfeife. Es besitzt einen zwei gekrümmte Abschnitte und einen zylindrischen Mittelteil aufweisenden Strömungskanal, der die schlitzförmige Gaseintrittsöffnung 2 mit der ebenfalls schlitzförmigen Gasaustrittsöffnung 3 verbindet. Das planare Sensorelement 4 ist in Strömungsrichtung gesehen hinter einem Metallsteg 5 angeordnet und in einer nicht dargestellten Bohrung des Gehäuses 1 befestigt. Da der Abstand zwischen dem Steg 5 und dem Sensorelement 4 nur wenige Millimeter beträgt, können sich im Zwischenraum keine Wirbel ausbilden. Im Mittelteil des Kanals strömt das Meßgas daher weitgehend laminar und parallel zur Oberfläche der gasempfindlichen Metalloxidschicht 6. Um die Strömung auch im hinteren Teil des Kanals wirbelfrei zu halten, ist der in die Gasaustrittsöffnung 3 mündende Endabschnitt gekrümmt ausgeführt. Hier erfährt der Gasstrom eine Ablenkung um annähernd 90°, so daß das aus dem Gehäuse 1 entweichende und das in das Gehäuse eintretende Meßgas in dieselbe Richtung strömen.

Der in die Gaseintrittsöffnung 2 mündende Abschnitt des Kanals ist ebenfalls gekrümmt und lenkt den Gasstrom um annähernd 90° in Richtung des Sensorelements 4 um. Da die im Meßgas vorhandenen schwereren Teilchen der Kanalkrümmung aufgrund ihrer Trägheit nicht folgen können, treffen sie im Bereich 7 auf die Kanalwand und bleiben dort haften. Die im abgelenkten Gasstrom eventuell noch mitgeführten leichteren Teilchen prallen auf den in Strömungsrichtung unmittelbar vor dem Sensor 4 angeordneten Metallsteg 5 bzw. werden aufgrund der sich hinter dem Steg 5 ausbildenden Gasströmung parallel am Sensorelement 4 vorbeigeführt.

Um eine weitgehend wirbelfreie Umströmung des Sensorelements 4 zu gewährleisten, sollte dieses einen planaren Aufbau besitzen und in der in Fig. 1 dargestellten Weise bezüglich des Steges ausgerichtet sein. Das Meßgas strömt insbesondere dann parallel zur gassensitiven Schicht 6, wenn die Stirnflächen des Steges 5 und des Sensorelements 4 annähernd dieselbe Größe aufweisen und der Steg 5 als ein sich in Strömungsrichtung verjüngender Körper ausgeführt ist.

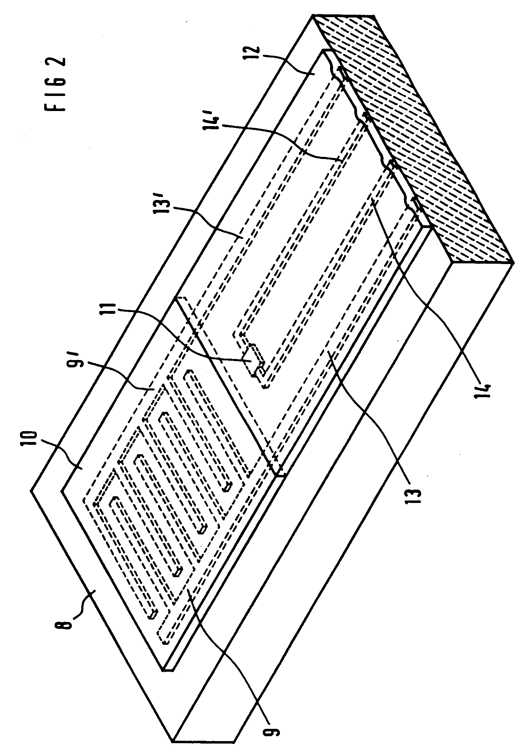

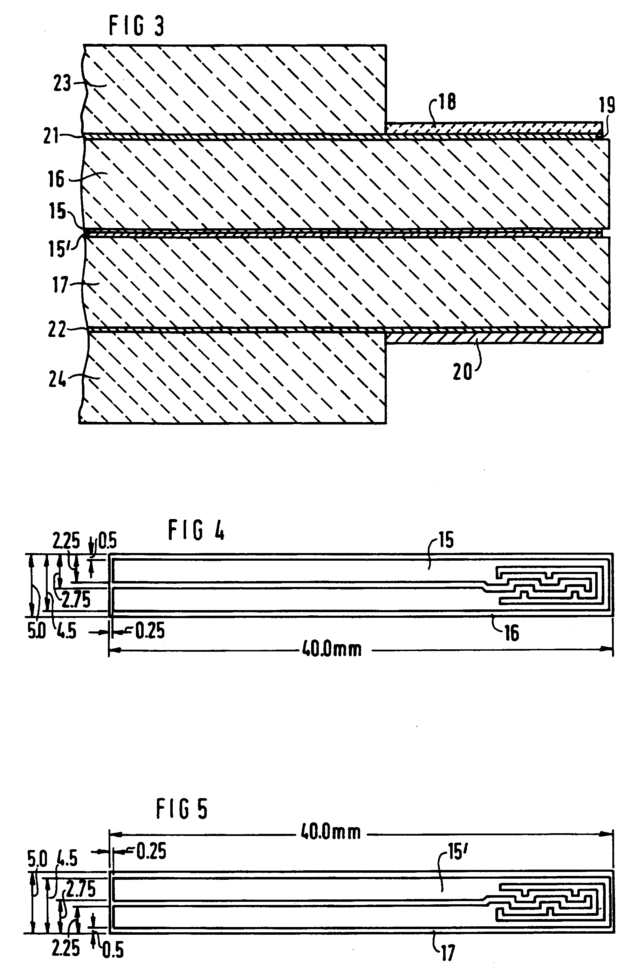

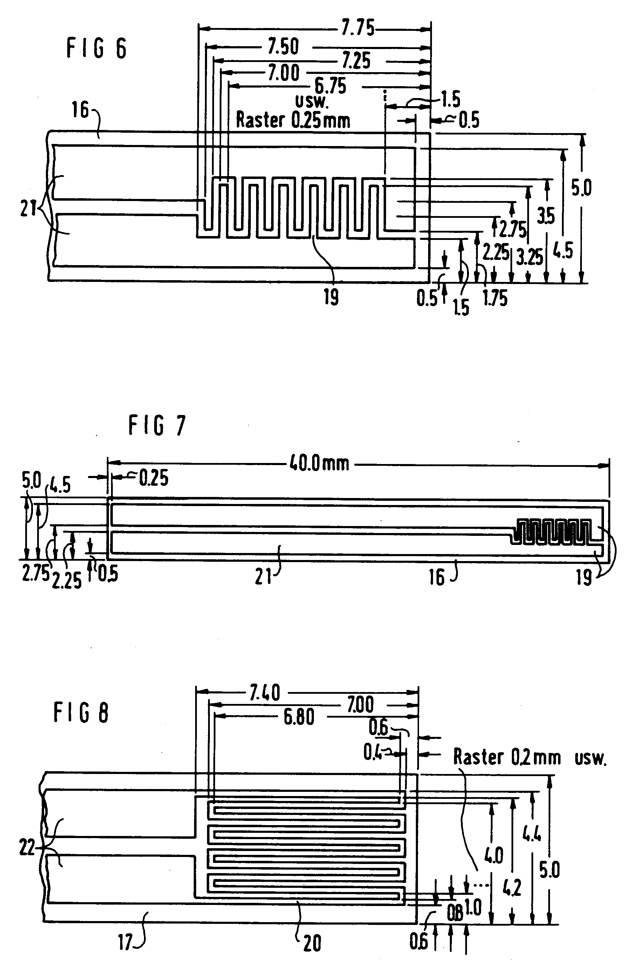

Der in Fig. 2 schematisch dargestellte Sensorelement zur Messung des Partialdrucks von Sauerstoff ähnelt im Aufbau den aus der EP-A-0 464 243 und 0 464 244 bekannten Detektoren. Er besitzt ein beispielsweise aus Magnesium-, Silizium- oder Aluminiumoxid bestehendes Substrat 8, auf dessen Oberfläche zwei eine Interdigitalstruktur bildende Platinelektroden 9 und 9', eine diese Elektroden bedeckende und etwa 1 bis 2 µm dicke Strontium- oder Bariumtitanatschicht 10 sowie ein Temperaturfühler 11 angeordnet sind. Die mit 12 bezeichnete Passivierungsschicht aus Glas oder Siliziumoxid schirmt die den Elektroden 9 und 9' und dem Temperaturfühler 11 jeweils zugeordneten Anschlußleitungen 13 und 13' bzw. 14 und 14' von dem im Meßgas vorhandenen Sauerstoff ab. Als Heizelement findet eine auf der Rückseite des Substrats 8 angeordnete Widerstandsschicht aus Platin Verwendung, die beispielsweise die in den Figuren 4 oder 5 dargestellte Struktur aufweisen kann.

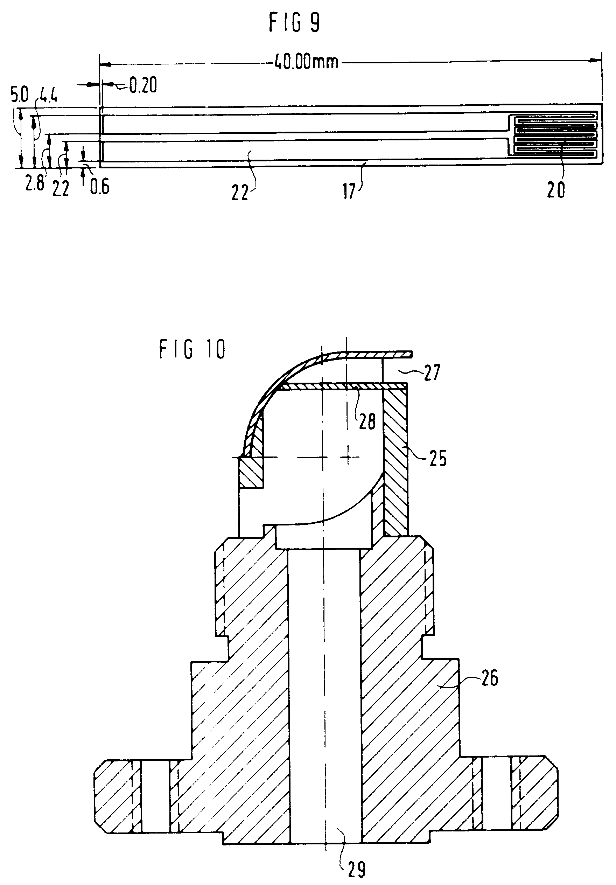

Die Fig. 3 zeigt einen Schnitt durch den Kopf eines für den Einbau in das Pfeifengehäuse 1 besonders geeigneten Sensorelements. Bei dieser Ausführungsform dienen die mit 15 bzw. 15' bezeichneten und in den Figuren 4 und 5 dargestellten Platinschichten als Heizelement. Sie werden mittels eines Siebdruckverfahrens auf die Al₂O₃-Substrate 16 und 17 aufgebracht. Auf den den Platinschichten 15 und 15' jeweils gegenüberliegenden Flächen der Substrate 16 bzw. 17 sind die sauerstoffsensitive SrTiO₃ oder BaTiO₃-Schicht 18 und die Interdigitalelektroden 19 bzw. der ebenfalls aus Platin bestehende Temperatursensor 20 sowie deren Anschlußelektroden 21 bzw. 22 angeordnet. Vorteilhafte Geometrien für die das aufgesputterte Metalloxid 18 kontaktierenden Elektroden 19 und den Temperatursensor 20 finden sich in den Figuren 6 und 7 bzw. 8 und 9, wobei die Figuren 6 und 8 jeweils die Elektrodenstruktur im Bereich des Sensorkopfes zeigen. Die in Fig. 3 mit 23 und 24 bezeichneten Al₂O₃-Schichten sollen die den Interdigitalelektroden 19 und dem Temperatursensor 20 jeweils zugeordneten Anschlußleitungen 21 bzw. 22 vom Sauerstoff des den Sensor umströmenden Meßgases abschirmen.

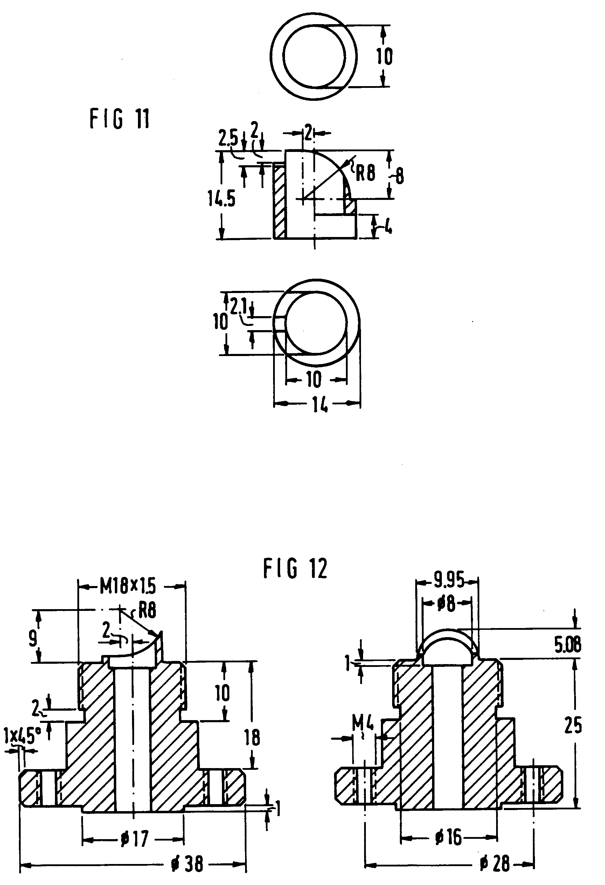

Das in Fig. 10 dargestellte Sensorgehäuse besteht aus zwei Teilen 25/26, wobei der beispielsweise aus Inconel gefertigte und die Gaseintrittsöffnung 27 und den Steg 28 enthaltende Gehäusekopf 25 auf dem mit einer Bohrung 29 zur Aufnahme des Sensorelements ausgestatteten Grundkörper 26 befestigt ist. Vor dem Verschweißen der beiden in den Figuren 11 und 12 im Schnitt dargestellten Teilen 25 und 26 wird das an den Außenflächen mit einem Keramikkleber bestrichene Sensorelement in die Bohrungen 29 des Grundkörpers 26 geschoben. Hierbei ist darauf zu achten, daß im Bereich des Sensorkopfes keine Verunreinigungen durch den Keramikkleber auftreten und die Klebestellen formgleich mit den Rundungen des Gehäusekopfes 25 abschließen. Die Aushärtung des Keramikklebers erfolgt während eines etwa 20 Minuten dauernden Tempervorgangs bei 200 °C.

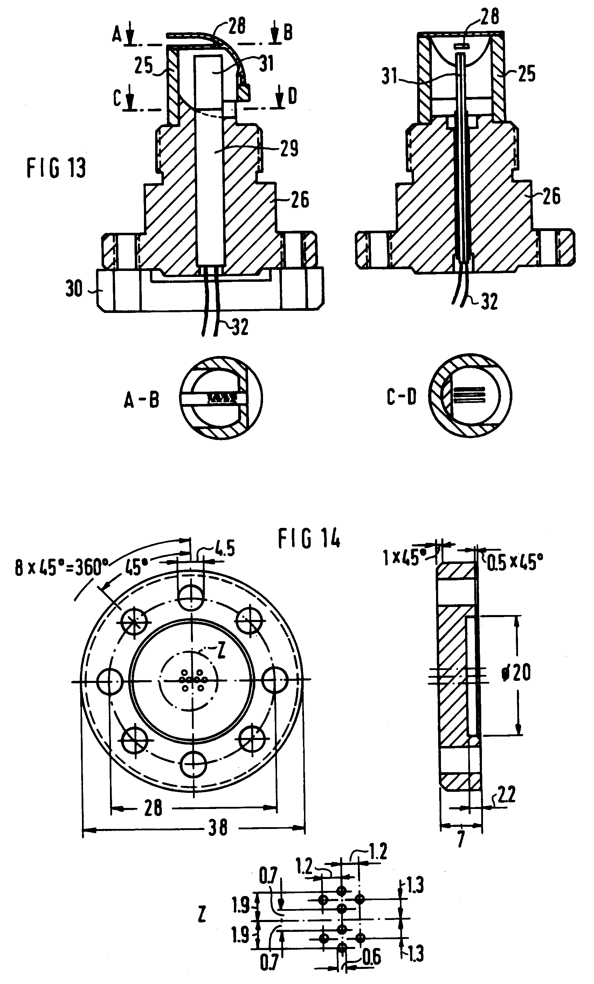

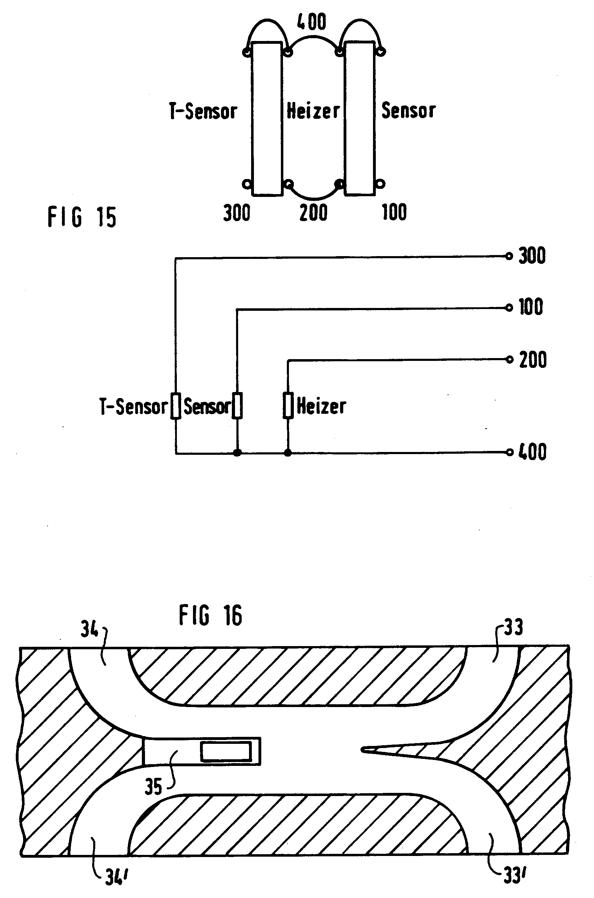

Fig. 13 zeigt den fertig montierten Gassensor. Dargestellt ist auch die Keramik-(Makor-)-Platte 30, die die verklebte Bohrung 29 des unteren Gehäuseteils 26 abschließt (s. auch Fig. 14). Sie enthält insgesamt acht Durchführungen, durch die man die zur Kontaktierung des Sensorelements 31 erforderlichen Platindrähte 32 nach außen führt.

Um die Anzahl der elektrischen Anschlüsse von acht auf vier zu verringern, werden die Massen des Sensorelements, des Temperatursensors und der beiden Heizelemente in einem Anschluß 400 zusammengefaßt (s. Fig. 15). Während die Heizspannung zwischen den Anschlüssen 200 und 400 anliegt, kann man das den Sauerstoffpartialdruck repräsentierende Sensorsignal zwischen den Anschlüssen 100 und 400 und den Widerstand des Temperatursensors zwischen den Anschlüssen 300 und 400 abgreifen. Zur Isolation der Platindrähte werden diese beispielsweise in eine Glaskeramik aus Silizium-, Magnesium- oder Boroxiden eingeschmolzen.

Die Erfindung ist selbstverständlich nicht auf die beschriebenen Ausführungsbeispiele beschränkt. So ist es ohne weiteres möglich, mehrere, vorzugsweise symmetrisch zur Längsachse des Sensorgehäuses angeordnete Gaseintritts- und Gasaustrittsöffnungen 33, 33' bzw. 34, 34' vorzusehen und diese durch gekrümmte Seitenkanäle mit dem das Sensorelement 35 aufnehmenden Hauptkanal zu verbinden (s. Fig. 16).

Auch ohne Verwendung eines Steges ist das Sensorelement sehr gut gegen Ablagerungen geschützt. Aufgrund der weitgehend wirbelfreien Strömung im Kanal besitzen die im umgelenkten Meßgas noch mitgeführten leichteren Teilchen keine oder nur eine sehr kleine transversale Geschwindigkeitskomponente in Richtung der sensitiven Schicht. Sie werden daher sehr schnell am Sensorelement vorbeigeführt und mit dem Meßgas aus dem Gehäuse transportiert. Eine in bekannten Systemen häufig beobachtete Ansammlung von Teilchen im Gehäuseinnern kann nicht stattfinden.

Das oben beschriebene Prinzip des Schutzes eines Sensorelements vor den in einem Gasstrom mitgeführten Teilchen läßt sich selbstverständlich auch bei Flüssigkeiten anwenden.

## Patentansprüche

1. Sensor mit einem Sensorelement (4) und einem das Sensorelement (4) aufnehmenden Gehäuse (1), wobei das Gehäuse (1) mit einer Eintrittsöffnung (2) und einer Austrittsöffnung (3) für ein gasförmiges oder flüssiges Medium ausgestattet ist,
**dadurch gekennzeichnet,**
- daß ein eine Krümmung aufweisender Strömungskanal die Eintrittsöffnung (2) mit der Austrittsöffnung (3) verbindet und
- daß das Sensorelement (4) im Kanal in Strömungsrichtung gesehen hinter der Krümmung angeordnet ist.

2. Sensor nach Anspruch 1,
**dadurch gekennzeichnet,**
daß der Strömungskanal s-förmig gekrümmt ist.

3. Sensor nach Anspruch 1 oder 2,
**dadurch gekennzeichnet,**
daß der Strömungskanal einen in die Eintrittsöffnung (2) mündenden ersten Abschnitt und einen in die Austrittsöffnung (3) mundenden zweiten Abschnitt aufweist und daß das Sensorelement (4) in einem den jeweils gekrümmten ersten und zweiten Kabelabschnitt verbindenden Mittelteil angeordnet ist.

4. Sensor nach Anspruch 3,
**dadurch gekennzeichnet,**
daß der erste und/oder der zweite Abschnitt des Strömungskanals jeweils eine Krümmung von etwa 90° aufweisen.

5. Sensor nach Anspruch 3 oder 4,
**dadurch gekennzeichnet,**
daß das den den ersten und den zweiten Kanalabschnitt verbindende Mittelteil einen kreisförmigen Querschnitt aufweist.

6. Sensor nach einem der Ansprüche 1 bis 5,
**dadurch gekennzeichnet,**
daß das Sensorelement (4) in einem weitgehend laminar durchströmten Bereich des Strömungskanals angeordnet ist.

7. Sensor nach einem der Ansprüche 1 bis 6,
**dadurch gekennzeichnet,**
daß das eine planare Struktur aufweisende Sensorelement (4) derart ausgerichtet ist, daß das Medium Parallel zur Oberfläche des Sensorelements (4) strömt.

8. Sensor nach einem der Ansprüche 1 bis 7,
**gekennzeichnet durch**
eine in Strömungsrichtung unmittelbar vor dem Sensorelement (4) angeordnete Abschirmung (5).

9. Sensor nach Anspruch 8,
**dadurch gekennzeichnet,**
daß die vom Medium angeströmten Stirnflächen der Abschirmung (5) und des Sensorelements (4) annähernd gleich groß sind.

10. Sensor nach Anspruch 7 oder 8,
**dadurch gekennzeichnet,**
daß die Abschirmung (5) sich in Strömungsrichtung verjüngt.

11. Sensor nach einem der Ansprüche 1 bis 10,
**dadurch gekennzeichnet,**
daß das Gehäuse (1) aus einem oberen und einem unteren Teil besteht, wobei der obere Teil die Eintrittsöffnung (2), den ersten Abschnitt des Strömungskanals und ggf. die Abschirmung (5) enthält und das Sensorelement (4) am unteren Teil des Gehäuses befestigt ist.

12. Sensor nach einem der Ansprüche 1 bis 11,
**dadurch gekennzeichnet,**
daß die Eintritts- und die Austrittsöffnung (2, 3) jeweils schlitzförmig ausgebildet sind.

13. Sensor nach einem der Ansprüche 1 bis 12,
**dadurch gekennzeichnet,**
daß das Gehäuse (1) mehrere jeweils in den Strömungskanal mündende Eintritts- und Austrittsöffnungen (33, 33', 34, 34') besitzt.

14. Verwendung eines Sensors nach einem oder mehreren der vorhergehenden Ansprüche als λ-Sonde.
